# DEMANDE DE BREVET EUROPEEN

(11) **EP 2 730 651 A1**
(43) Date de publication de la demande: **14.05.2014**
(21) Numéro de dépôt: 12306384.4
(22) Date de dépôt: 08.11.2012
(51) Int. Cl.: C12N 9/16, C12P 19/04

(54) **Kappa carraghénane sulfatase, procédé de fabrication et utilisation**

(71) Demandeur: Centre National de la Recherche Scientifique (CNRS), 75794 Paris Cedex 16 (FR); Université Pierre et Marie Curie - Paris 6, 75005 Paris (FR)
(72) Inventeur: Helbert, William, 38410 Saint Martin d'Uriage (FR); Prechoux, Aurélie, 29370 Elliant (FR)
(74) Mandataire: Novagraaf Technologies

(57) **Abrégé**

La présente invention se rapporte notamment à des protéines, à des séquences d'acides nucléiques codant pour ces protéines, à des vecteurs comprenant ces séquences codantes, à un procédé de fabrication de ces protéines, ainsi qu'à un procédé de transformation de kappa-carraghénanes en béta-carraghénanes utilisant ces protéines. En particulier, elle concerne la protéine de séquence SEQ ID n°1 ou 2. La présente invention trouve des applications dans la valorisation des bio-ressources naturelles constituées par les organismes et les microorganismes comprenant des carraghénanes.

## Description

### Domaine technique

La présente invention se rapporte notamment à des protéines, à des séquences d'acides nucléiques codant pour ces protéines, à des vecteurs comprenant ces séquences codantes, à un procédé de fabrication de ces protéines, ainsi qu'à un procédé de conversion de kappa-carraghénanes utilisant ces protéines. La présente invention se rapporte également aux carraghénanes obtenus par ledit procédé de conversion.

La présente invention trouve notamment des applications dans la valorisation des bio-ressources naturelles constituées par les organismes et les microorganismes. En particulier, elle trouve des applications en laboratoire, ainsi que dans l'agro-alimentaire, dans le domaine de la cosmétique et dans le domaine des médicaments et formulations pharmaceutiques où les produits issus de la conversion de kappa-carraghenane sont valorisables.

Dans la description ci-dessous, les références entre crochets [ ] renvoient à la liste des références présentée à la fin du texte.

### Etat de la technique

Les carraghénanes définissent ainsi des familles de galactanes sulfatés isolés de la matrice extra-cellulaire des algues rouges marines.

La grande diversité des structures chimiques des carraghénanes et leur hybridité naturelle confèrent à chaque extrait d'algues des propriétés fonctionnelles caractéristiques. En outre, ces polysaccharides anioniques ont des propriétés rhéologiques uniques et sont utilisés comme agents de texture en industrie agro-alimentaire, parapharmaceutique et cosmétique

Les carraghénanes sont extraits des algues rouges telles que *Furcellaria lumbricallis, Eucheuma denticulatum, Kappaphycus alvarezii* et *Chondrus crispus.* Ce sont les polysaccharides marins les plus exploités, environ 50 000 tonnes/an devant les alginates environ 26 500 tonnes/an et les agars environ 9 600 tonnes/an. En 2010, les carraghénanes, représentant près de 561 millions de de dollars de chiffre d'affaire, se situaient en quatrième position du marché des hydrocolloïdes, après l'amidon, la gélatine et les pectines (Seinsun, Overview of the Food Hydrocolloids Market. Gums and Stabilisers for the Food Industry 16, 1-8 2011 [1]).

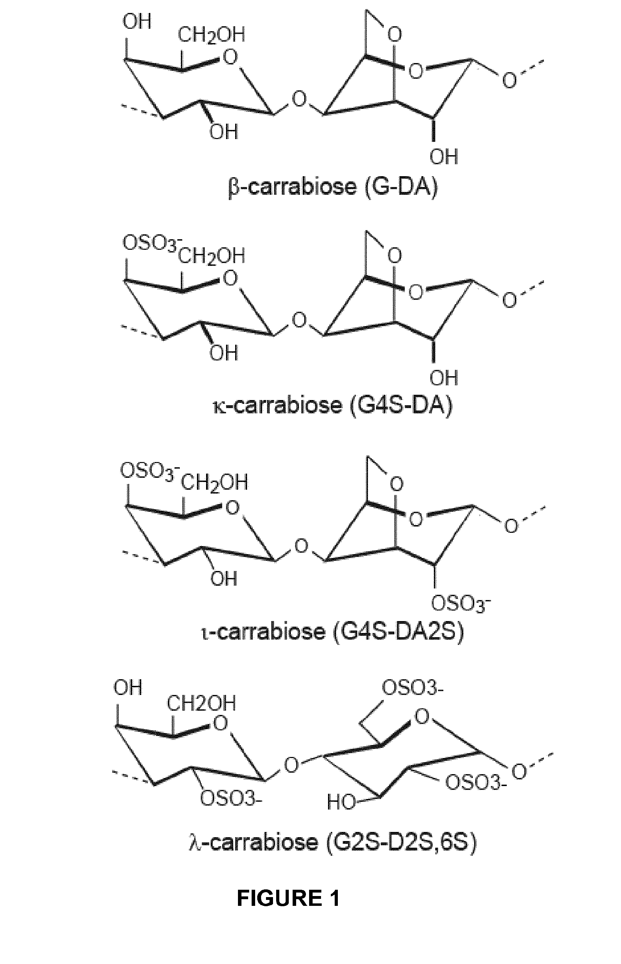

Les carraghénanes sont composés d'un enchaînement de D-galactoses liés alternativement par des liaisons α(1-3) et β(1-4). Les unités de répétition sont donc des disaccharides appelés unités carrabioses. Les unités de répétition sont classées en fonction de la présence ou non d'un pont 3,6 anhydro sur le résidu galactose lié en α(1-3) et par leur taux de sulfatation. Par exemple, le β-carrabiose se caractérise par l'absence de groupement sulfate alors que le κ-, le 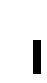 - et le λ-carrabiose, possèdent un, deux ou trois sulfates par unité disaccharidique, respectivement (Figure 1).

Les κ- et 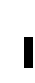 -carraghénanes sont composés principalement, mais pas uniquement, des motifs idéaux κ- et 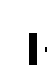 -carrabioses. D'autres motifs comme les précurseurs biosynthétiques (µ- et v-carraghénanes) ou bien des structures désulfatées (béta-carrabiose), méthylées ou pyruvatées peuvent également être présentes dans la chaîne de polysaccharides. Par conséquent, en fonction des sources d'algues et des conditions d'extraction on peut observer un large spectre de structures de carraghénanes.

Les κ- et 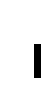 -carraghénanes ont la propriété de former des gels iono-et thermo-dépendants. Le κ-carraghénane va former des gels rigides en présence de potassium alors que le 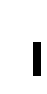 -carraghénane forme des gels souples et élastiques en présence de calcium.

Le furcellaran désigne le carraghénane extrait de l'algue rouge *Furcellaria lumbricallis* (Danish moss). Ce carraghénane est composé d'unités κ- et β-carrabioses distribuées aléatoirement le long de la chaine du polysaccharide. Cette distribution se distingue d'une organisation plutôt en bloc des unités κ- et β-carrabioses observée dans le cas des κ-/β-carraghénanes d'*Eucheuma gelatinae* et de *Tichocarpus crinitus* (Greer and Yaphe, 1984, Characterization of hybrid (beta-kappa-gamma) carrageenan from *Eucheuma gelatinae* agardh,j. (rhodophyta, solieriaceae) using carrageenases, infrared and 13C-nuclear magnetic-resonance spectroscopy. Bot Mar 27: 473-478 [2]; Correc et al., 2012 Comparison of the structures of hybrid κ-/β-carraghenans extracted from Furcellaria lumbricalis and Tichocarpus crinitus. Carbohydr. Polym. (2012) 88, 31-36 [3]) Le furcellaran permet de former des gels qui présentent des propriétés de rigidité proches de celle du κ-carraghénane mais en raison du plus faible taux de sulfatation, les concentrations salines nécessaires à la gélification sont plus faibles (Bjerre-Petersen et al. 1973 [4] ; Zhang et al. Cation specific and cation binding to low sulfated carrageenans. Carbohydr. Polym. 23, 105-110. 1994 [5]). Toutefois, de part la diminution de *F. lumbricallis,* causé par son exploitation intensive, les ressources en furcellaran sont désormais limitées et son utilisation limitée.

Il existe donc un réel besoin de trouver de nouveaux moyens qui permettraient d'obtenir des carraghénanes hybrides et/ou uniques à partir des algues cultivées. Il existe également un réel besoin de trouver un nouveau moyen permettant d'obtenir un nouveau procédé, plus économique permettant d'obtenir des carraghénanes hybrides et/ou unique permettant d'ouvrir de nouvelles perspectives quant à l'exploitation et la valorisation de la biomasse d'algues rouges, notamment dans les domaines cosmétiques, agro-alimentaires et médicales.

Il existe également un réel besoin de trouver des moyens permettant de produire des carraghénane tel que le furcellaran.

### Exposé de l'invention

La présente invention a précisément pour but de répondre à ce besoin en fournissant des protéines qui transforme les Kappa-carraghénannes en béta-carraghénannes. L'étude des modalités de reconnaissance des enzymes de la présente invention réalisée par les inventeurs démontre leur activité 4S-kappa-carraghénane-sulfatase.

La présente invention a pour objet une kappa carraghénane sulfatase extraite du microorganisme *Pseudoalteromonas atlantica* T6c déposé sous le numéro ATCC T6c/BAA-1087 à American Type Culture Collection (ATCC).

La présente invention a également pour objet une kappa carraghénane sulfatase extraite du microorganisme *Pseudoalteromonas Atlantica* T6c déposé sous le numéro ATCC T6c/BAA-1087 à American Type Culture Collection (ATCC) de séquence SEQ ID n°1 ou 2 du listage de séquences annexé, à savoir le peptide de séquence :
VPKNTAVAASKAAPTKKPNVLFISIDDLRPELGSYGSDIAITPNLDA LAKRGVQFNQAHAQQAICGPSRASILTGIRPDSLDVHHNYQLFRHKLEDV VTLPQHFANNGYEAFYVGKIFHHTDKDEALSWNVEPAYHKLPPGLAKPK RYALKANQKIQQDNRKAMFAKYGEQAKFGLGSGPAYESADVPDTAYHD GYNTELAIATMKEQLNNRDKPLFIGFGMMKPHLPWIAPQKYWDLYNPDDI TLAENDTAPIDGAAMGLHASFELRTFSNIPKKGPIAPTLARTLKHAYLANIS YVDAQIGKMLAALEEQGILDNTIVIVWSDHGWHLGEMGIWGKATNYDIAT RVPLIIATPDMKKDIQGQKTDALVELVDIYPTLSALAGLNNLPQWEGQSMV PLLTNPAHSWKPAVFSQFPTPALREWGAYPLRSGMRETYFGPLIKRVEQ RIKDQQKDKWDRELFEQHLMGNAIRTERYRLVAWQDTRLAKNSRPLYLE LYDHQHDPNESINVAKQHPKQVAKLLKQLYAGWQISKASLREPSI AGQTR (SEQ ID n°1), ou de séquence :
QPNIVFLFSDDAGYADFGFQGSETMKTPNLDQLASEGVRFTQGY VSDSTCGPSRAGIMTGRYQQKFGYEEINVPGYMSEHSAIKGAEMGIPLD EVTMGDYMKSLGYRTAFYGKWHLGGTDELHPMHRGFDEFYGFRGGDR SYWAYEVNAPERKSAVFTDKKLEHGIDQFQEHEGYLTDVLAEKANQFIEK APDKPFFIFLSFNAVHTPMEATPEDLAKFPQLKGKRKEVAAMTLALDRAS GAVLNKLKELGLEDDTLVVFSNDNGGPTDKNASSNYPLAGTKSNFLEGGI RVPFLVKWPAKLAAGKVYDKPVSTLDLLPTFFKAGGGEEVMSELDGVDL MPYITGQNNKAPHESMYWKKETRAAIRQGDWKLLRFPDRPAELYNLAND IGEQHNLAAQEPERVKQMYKDFFSWEMTLERPLWLLKRQFEEYDLNRM DKYRVPKRLE (SEQ ID n°2).

Selon l'invention, la protéine objet de l'invention peut comprendre en outre, à son extrémité N-terminale, une séquence signal ou séquence d'adressage. Cette séquence signal peut être une des séquences signal connues de l'homme du métier pour que la protéine, lorsqu'elle est synthétisée dans une cellule hôte, soit dirigée vers un organite ou une zone particulière de la cellule hôte. Il peut s'agir par exemple d'une séquence signal trouvée dans les sites spécialises dans la prédiction de peptides signaux par exemple http://www.cbs.dtu.dk/services/SignaIP/ [6] ou encore http://bmbpcu36.leeds.ac.uk/prot-analysis/Signal.html [7] Il peut s'agir par exemple la séquence SEQ ID n°3 ou 4 du listage de séquences annexé, à savoir la séquence MRKYLIMLLILAGCSPVLAD (SEQ ID n°3) ou MKSVFHRVVA ALSISVACTSLSYAK (SEQ ID n°4). Cette séquence signal peut être clivée après synthèse de la protéine ou non.

Avantageusement, les inventeurs ont noté que les séquences signal ne gênent pas et que leurs clivages n'est pas nécessaire pour l'expression, l'activité ou le fonctionnement de la protéine, par exemple la protéine est surexprimée sans son peptide signal.

La présente invention se rapporte également aux acides nucléiques codant pour la kappa carraghénane sulfatase extraite du microorganisme *Pseudoalteromonas atlantica* T6c déposé sous le numéro ATCC T6c/BAA-1087 à American Type Culture Collection (ATCC).de la présente invention.

La présente invention se rapporte également aux acides nucléiques codant pour la kappa carraghénane sulfatase de la présente invention notamment pour la protéine de séquence SEQ ID n°1 ou 2. L'acide nucléique de la présente invention peut être toute séquence codant pour le peptide de séquence SEQ ID N° 1 ou 2 compte tenu de la dégénérescence du code génétique. Il peut s'agir par exemple d'un acide nucléique comprenant ou constitué de la séquence SEQ ID n°5 du listage de séquences annexé, à savoir l'acide nucléique de séquence : 
GTACCAAAGAACACTGCCGTAGCTGCTAGCAAAGCTGCACCAACTAA
AAAGCCAAATGTATTATTTATCTCGATTGATGACTTGCGCCCTGAGCT
GGGCAGTTACGGTTCTGATATTGCCATAACACCCAATTTAGATGCTTT
GGCGAAGCGCGGCGTACAGTTTAATCAAGCTCATGCACAGCAAGCGA
TATGTGGGCCATCTCGCGCCAGCATATTAACAGGCATTCGACCTGATT
CACTTGATGTACATCATAATTATCAATTGTTCAGACATAAACTTGAGGA
CGTGGTTACCCTGCCTCAGCATTTTGCTAACAATGGTTATGAAGCATT
TTACGTGGGTAAAATCTTCCATCACACCGACAAAGACGAAGCACTGTC
TTGGAATGTTGAGCCCGCTTATCACAAGCTACCTCCTGGGTTAGCAAA
ACCCAAACGTTATGCATTAAAAGCCAACCAAAAAATTCAGCAAGATAA
CCGTAAGGCTATGTTCGCCAAGTACGGTGAACAAGCTAAATTTGGCTT
AGGTAGTGGGCCAGCATATGAATCTGCAGACGTGCCAGATACGGCCT
ACCACGACGGTTATAACACCGAGCTTGCCATTGCTACGATGAAAGAG
CAGTTGAATAATCGTGATAAGCCTTTGTTTATCGGATTTGGATGATG
AAGCCGCACTTGCCATGGATCGCGCCGCAAAAATATTGGGACCTTTA
TAACCCAGATGACATTACTCTAGCTGAGAACGACACAGCGCCTATTGA
TGGCGCAGCAATGGGTTTACACGCATCTTTTGAACTTCGCACTTTTTC
CAATATTCCCAAAAAAGGGCCTATAGCACCGACTCTAGCGCGCACAC
TCAAGCATGCTTATTTAGCCAACATCAGTTATGTGGACGCGCAGATTG
GGAAAATGCTTGCAGCCCTTGAAGAGCAAGGGATCTTAGATAATACCA
TCGTGATCGTGTGGAGTGACCATGGCTGGCACTTAGGCGAAATGGGT
ATCTGGGGCAAAGCCACGAACTACGATATAGCCACCCGAGTTCCGCT
TATTATTGCCACGCCAGATATGAAAAAAGACATTCAAGGGCAAAAAC
AGATGCTCTGGTCGAACTGGTCGATATTTATCCGACGTTGTCAGCACT
TGCAGGCTTGAATAACTTACCCCAATGGGAAGGGCAAAGCATGGTGC
CGCTTTTGACCAATCCTGCTCACTCTTGGAAACCGGCTGTGTTTAGTC
 
AATTTCCTACGCCAGCATTGAGAGAATGGGGGGCTTATCCGCTGCGT
TCTGGTATGCGGGAAACTTACTTCGGGCCGCTTATTAAACGAGTTGAA
CAGCGCATCAAAGACCAACAAAAAGACAAATGGGATCGAGAACTATTT
GAACAACACTTGATGGGCAATGCTATACGTACCGAGCGATATCGGCT
GGTGGCTTGGCAAGATACCCGTTTAGCTAAGAATAGCCGTCCTTTGTA
CTTAGAGTTGTATGATCATCAACATGATCCTAACGAAAGCATCAATGTT
GCCAAACAACACCCAAAACAAGTGGCCAAACTACTCAAACAACTCTAT
GCGGGTTGGCAAATCAGTAAAGCGTCATTAAGAGAACCCTCAATCGC
AGGACAAACACGATGA (SEQ ID n°5),
 ou l'acide nucléique de séquence :

CAACCGAATATCGTTTTTCTGTTTTCCGATGATGCAGGGTATGC
TGACTTTGGTTTTCAAGGCAGCGAAACAATGAAAACACCCAATTTGGA
TCAGCTTGCTAGCGAAGGTGTTAGATTTACCCAAGGTTATGTGTCGGA
CTCTACTTGCGGCCCATCTCGGGCGGGCATCATGACAGGGCGCTAC
CAGCAAAAATTCGGTTACGAAGAAATAAACGTGCCGGGTTATATGAGT
GAGCACTCTGCTATAAAGGGGCAGAAATGGGGATCCCGTTAGATGA
AGTCACTATGGGTGACTACATGAAATCTCTTGGGTATCGCACGGCGTT
CTACGGTAAATGGCACTTAGGTGGTACGGATGAATTGCACCCTATGC
ACCGTGGCTTCGACGAATTTTATGGCTTTCGCGGCGGCGATCGCAGC
TACTGGGCCTATGAAGTCAATGCCCCTGAGCGCAAATCTGCCGTATTT
ACTGACAAAAAACTTGAACACGGTATTGATCAATTTCAAGAGCACGAA
GGGTATTTGACCGATGTGTTAGCCGAGAAAGCCAATCAATTTATTGAA
AAAGCGCCTGATAAACCGTTCTTTATTTTCTTATCATTTAACGCAGTGC
ATACACCAATGGAAGCCACGCCAGAAGATTTAGCGAAGTTTCCTCAGT
TAAAAGGCAAGCGCAAGGAAGTTGCAGCCATGACCCTCGCACTTGAT
AGAGCCAGCGGAGCAGTGCTAAACAAACTCAAGGAGCTGGGTTTGGA
AGATGACACTCTTGTGGTGTTCAGCAACGACAATGGCGGCCCTACGG
ATAAAAACGCCTCATCTAACTACCCATTAGCAGGGACTAAATCAAACT
TTTTGGAAGGGGGAATACGCGTGCCTTTTCTGGTTAAATGGCCTGCTA
AGCTAGCTGCTGGAAAGGTATACGATAAACCCGTCAGTACGCTCGAC
TTATTACCCACATTCTTTAAAGCGGGTGGCGGTGAAGAGGTGATGTCT

GAATTGGATGGCGTGGATTTGATGCCTTATATAACAGGCCAAAATAAC
AAGGCACCTCACGAGAGCATGTACTGGAAAAAGAAACTCGCGCAGC
TATTCGCCAAGGAGATTGGAAGTTATTGCGCTTCCCAGACCGTCCTG
CAGAATTGTATAACCTTGCTAATGACATTGGTGAACAACATAACTTAGC
CGCCCAAGAGCCTGAGCGTGTTAAGCAAATGTACAAAGATTTCTTCAG
TTGGGAAATGACACTAGAGCGCCCATTATGGTTGCTTAAGCGCCAGTT
TGAGGAATACGATTTAAATCGCATGGACAAGTACCGCGTACCAAAACG
CTTAGAATAA (SEQ ID n°6),

Selon l'invention l'acide nucléique codant pour la protéine de la présente invention peut comprendre en outre, à son extrémité 5', la séquence SEQ ID n° 7 ou 8 du listage de séquences annexé, à savoir un acide nucléique de séquence : 
ATGCGTAAATATTTAATAATGTTACTGATCTTGGCTGGTTGTTCA
CCTGTGCTTGCTGAT (SEQ ID n°7),
 ou un acide nucléique de séquence :

ATGAAATCAGTTTTTCATCGCGTTGTAGCAGCGCTTTCTATCAG
TGTGGCGTGCACTTCGTTAAGTTACGCCAAG (SEQ ID n°8).

En d'autres termes, la présente invention se rapporte également à un acide nucléique isolé tel que défini ci-dessus.

La présente invention se rapporte également à un vecteur comprenant un acide nucléique codant pour la protéine de la présente invention, par exemple un acide nucléique de séquence SEQ ID n°5 ou 6 du listage de séquences annexé.

On entend par « vecteur » au sens de la présente invention, des vecteurs d'expression et/ou de sécrétion de séquences d'acide nucléique dans une cellule hôte déterminée. Ils peuvent par exemple être des vecteurs d'origine plasmidique ou virale, comportant, outre la séquence d'acide nucléique, les moyens nécessaires à son expression. Ces moyens peuvent par exemple inclure un promoteur, des signaux d'initiation et de terminaison de la traduction, ainsi que des régions appropriées de régulation de la transcription. Le vecteur d'expression peut également comprendre d'autres éléments tels qu'une origine de réplication, un site de clonage multiple, un enhanceur, un peptide signal qui pourra être fusionné en phase avec le polypeptide produit lors du clonage, et un ou plusieurs marqueurs de sélection. Le vecteur peut être un des vecteurs connus de l'homme du métier pour fabriquer des protéines par recombinaison génétique. Il est choisi en général notamment en fonction de l'hôte cellulaire choisi. Le vecteur peut être, par exemple pour une cellule de mammifère choisi parmi les vecteurs listés dans le catalogue http://www.promega.com/vectors/mammalian_express_vectors.htm **[8]** ou http://www.qiagen.com/pendantview/qiagenes.aspx?gaw=PROTQIAgenes 0807&gkw=mammalian+expression **[9]**, ou encore http://www.scbt.com/chap_exp_vectors.php?type=pCruzTM%20Expressio n%20Vectors **[10],** par exemple pour une cellule bactérienne, par exemple *Escherichia Coli* choisi parmi les vecteurs listés http://www.emdmillipore.com/life-science-research/vector-table-novagen-pet-vector-table/c_HdSb.s1O77QAAAEhPqsLdcab **[15]** et/ou http://fr-fr.invitrogen.com/site/fr/fr/home/Products-and-Services/Applications/Protein-Expression-and-Analysis/Protein-Expression.html**[16]**. Il peut s'agir par exemple du vecteur d'expression décrit dans le document WO 83/004261 **[11]**

Les acides nucléiques de la présente invention ou les vecteurs de la présente invention sont utilisables notamment pour la fabrication par recombinaison génétique de protéines de la présente invention. Aussi, la présente invention se rapporte également à une cellule hôte comprenant une séquence d'acide nucléique selon l'invention ou un vecteur selon l'invention.

La présente invention a également pour objet un procédé de production de kappa carraghénane sulfatase par une cellule hôte comprenant un acide nucléique codant pour ladite kappa carraghénane sulfatase et/ou un vecteur comprenant une séquence d'acide nucléique codant pour ladite kappa carraghénane sulfatase.

Selon l'invention, lorsque l'acide nucléique est dans une cellule hôte, il peut être isolée ou non.

On entend par « cellule hôte » au sens de la présente invention une cellule procaryote ou eucaryote. Des cellules hôtes couramment utilisées pour l'expression de cellules recombinantes incluent notamment des cellules de bactéries telles que *Escherichia coli* ou *Bacillus sp.,* des cellules de levures telles que *Saccharomyces cerevisiae,* des cellules de champignons tels que *Aspergillus niger,* des cellules d'insectes, et des cellules de mammifères (notamment humaine) telles que les lignées cellulaires CHO, HEK 293, PER-C6, etc... La transformation des cellules procaryotes et eucaryotes est une technique bien connue de l'homme du métier, par exemple la lipofection, l'électroporation, le choc thermique, ou des méthodes chimiques. En fonction de la cellule à transformer, l'homme du métier pourra aisément déterminer les moyens nécessaires à l'introduction et à l'expression de l'acide nucléique chez la cellule hôte choisie. Ainsi le vecteur d'expression et la méthode d'introduction du vecteur d'expression au sein de la cellule hôte seront sélectionnés en fonction de la cellule hôte choisie. La cellule hôte transformée par un vecteur d'expression ou un acide nucléique va exprimer le polypeptide correspondant de manière stable. L'homme du métier peut aisément vérifier que la cellule hôte exprime le polypeptide de manière stable, par exemple en utilisant la technique du Western blot.

La cellule hôte ou hôte cellulaire peut être tout hôte approprié pour la fabrication de kappa carraghénane sulfatase de la présente invention à partir des acides nucléiques ou des vecteurs de l'invention. Il peut s'agir par exemple *d'E. coli,* par exemple *E. coli* BL21, *E. coli* Origami (DE3) de *Pischia pastoris,* de *Saccharomyces cerevisiae,* de cellules d'insectes, par exemple un système cellules d'insectes-baculovirus (par exemple cellules d'insecte SF9 utilisant un système d'expression de baculovirus), de mammifères.

La présente invention se rapporte donc également à un procédé de fabrication de kappa carraghénane sulfatase de la présente invention comprenant la mise en culture une cellule hôte comprenant une séquence d'acide nucléique selon l'invention ou un vecteur selon l'invention.

Cette mise en culture se fait de préférence dans un milieu de culture permettant la croissance du microorganisme. Il peut s'agir par exemple milieu de culture liquide ZoBell, comme décrit dans le document ZoBell, CE 1941 Studies on marine bacteria. I. The cultural requirements of heterotrophic aerobes, J Mar Res 4, 41-75 **[12].**

Des conditions de culture utilisables pour la mise en oeuvre de la présente invention sont également décrites dans ce document. Le pH de culture est de préférence compris entre 7 et 9, de préférence pH 8. La température de culture est de préférence comprise entre 10 et 30°C, de préférence 18°C.

Le procédé de fabrication des protéines selon l'invention utilisant toute cellule hôte transformée pour une fabrication par recombinaison génétique conformément à la présente invention, peut comprendre en outre une étape de récupération des protéines selon l'invention. Cette étape de récupération ou d'isolement peut être réalisée par tout moyen connu de l'homme du métier. Il peut s'agir par exemple d'une technique choisie parmi une électrophorèse, un tamisage moléculaire, une ultracentrifugation, une précipitation différentielle, par exemple au sulfate d'ammonium, par ultrafiltration, une filtration sur membrane ou sur gel, un échange d'ions, une élution sur hydroxyapatite, une séparation par interactions hydrophobes, ou tout autre moyen connu. Il peut s'agir par exemple d'une étape utilisant un dispositif connu dans l'état de la technique, par exemple un système Akta Purifier (marque déposée). Un exemple de procédé d'isolement de kappa carraghénane sulfatase utilisables pour la mise en oeuvre de la présente invention est décrit ci-dessous.

La présente invention a également pour objet un procédé de transformation de kappa-carraghénanes en béta-carraghénanes comprenant l'utilisation d'un microorganisme déposé sous le numéro ATCC T6c/BAA-1087 à American Type Culture Collection (ATCC) et/ou une kappa-carraghénane sulfatase selon l'invention et/ou avec une cellule hôte comprenant un vecteur selon l'invention.

Selon l'invention, le procédé de transformation de kappa-carraghénanes en béta-carraghénanes peut comprendre avantageusement l'utilisation simultanée de la kappa carraghénane sulfatase de séquence SEQ ID n°1 du listage de séquences annexé et de la kappa carraghénane sulfatase de séquence SEQ ID n°2 du listage de séquences annexé.

La cellule hôte transformée, c'est-à-dire comprenant un vecteur selon l'invention, précitée utilisée pour une fabrication par recombinaison génétique conformément à la présente invention peut également être utilisée directement pour transformer des kappa-carraghénanes en béta-carraghénanes, dans leur milieu naturel ou en culture. Lorsqu'il s'agit d'une culture, il peut s'agir d'un système discontinu ou continu. On peut utiliser par exemple un réacteur de culture contenant un milieu de culture approprié au développement du microorganisme.

La présente invention a également pour objet les carraghénanes susceptibles d'être obtenus par le procédé de l'invention.

Selon un mode de réalisation particulier du procédé de l'invention, les carraghénanes obtenus possèdent des motifs béta-carrabioses dès lors que le carraghénane de départ possédait dans sa structure des motifs kappa-carrabioses. Par exemple, les motifs kappa-carrabioses du carraghénane extrait de l'algue rouge *Kappaphycus alvarezzi* peuvent être convertis en un carraghénane possédant de 100/0 à 0/100 de kappa/beta-carabiose. La réaction enzymatique peut être contrôlée et le taux de conversion de kappa-carrabiose en beta-carrabiose peut varier de 0 à 100%.

Avantageusement, les propriétés rhéologiques des carraghénanes obtenus peuvent être modulées en fonction du taux de conversion.

La présente invention permet donc avantageusement de transformer du kappa-carraghénane en béta-carraghénane et/ou en hybride kappa/béta-carraghénane.

La présente invention permet donc avantageusement de produire des analogues du furcellaran. En outre, la présente invention permet avantageusement de produire lesdits analogues de furcellaran tout en conservant les algues dont il est normalement extrait.

La présente invention permet également de fournir un procédé naturel permettant la production d'analogues du furcellaran. En outre, le procédé de l'invention fournit avantageusement un procédé de biotransformation de kappa-carraghénane. La présente invention permet également, avantageusement, d'éviter la mise en oeuvre de procédés chimiques, notamment de procédés chimiques de désulfatation couteux et pouvant être à l'origine de pollutions de l'environnement pour la production d'analogues du furcellaran et/ou de béta-carraghénanes.

D'autres caractéristiques et avantages apparaîtront encore à l'homme du métier à la lecture des exemples ci-dessous, donnés à titre illustratif et non limitatif, en référence aux figures annexées.

### Brève description des figures

- La figure 1 représente la structure chimique des motifs de répétition des quatre principaux carraghénanes (béta β, kappa κ, iota τ, et lambda λ) exploités industriellement.
- La figure 2 représente le schéma de réaction enzymatique catalysée par la 4S-kappa-carraghénane sulfatase.
- La figure 3 représente un gel d'électrophorèse (SDS-PAGE) coloré au bleu de Coomassie colloïdal de plusieurs fractions collectées après l'étape de chromatographie échangeuse d'anions (Q Sepharose Fast Flow). La protéine de 60 kDa est indiquée (1) et correspond à une 4S-kappa-carraghénane sulfatase.

- La figure 4 représente le spectre ¹H RMN du kappa-carraghénane (B) incubé avec la protéine purifiée de *Pseudoalteromonas atlantica* (Q15XH1) (A) correspondant à une kappa-carraghénane sulfatase
- La figure 5 représente la séquence en acides aminés de la protéine de *Pseudoalteromonas atlantica* T6c (Q15XH1). Les séquences peptidiques identifiées par spectrométrie de masse sont soulignées. En gras, la signature de la modification post-traductionnelle de la cystéine.
- La figure 6 représente l'environnement génomique du gène de la nouvelle 4S-kappa-carraghénane sulfatase (Q15XH1) dans *P. atlantica.*
- La figure 7 représente des diagrammes de suivie de la libération d'ions SO₃⁻.

La figure 7A représente un diagramme de libération du SO₃⁻ dans le milieu. L'ordonné représente la conductivité en micro Siemens (µS), l'abscisse le temps en minutes avec (+Enz) ou sans kappa-carraghénane sulfatase (-Enz).

La figure 7B est un diagramme en bâtons représentant la concentration des ions sulfate libérés dans le milieu d'incubation (ordonné) en fonction des conditions température 20°C (bâtons clairs) ou 37°C (bâtons grisés) et du substrat à savoir k- (k-carraghénane), k-/m- (hybride de kappa/mu-carraghénane) et oligo-k (oligo-kappa-carraghénane).

La figure 7C est un diagramme en bâtons représentant la concentration des ions sulfate libérés dans le milieu d'incubation (ordonné) en fonction de la concentration d'isopropyl β-D-1-thiogalactopyranoside (IPTG) (abscisse) utilisé lors de l'induction de la kappa-carraghénane sulfatase

La figure 7D est un diagramme en bâtons représentant la concentration des ions sulfate libérés dans le milieu d'incubation (ordonné) en fonction du temps d'induction (D) des cultures d'*E*. *coli.*
- La figure 8 représente la séquence en acides aminés de la protéine de *Pseudoalteromonas atlantica* T6c (Q15XG7).
- La figure 9 représente Spectres spectre ¹H RMN du kappa-carraghénane (A) incubé avec la protéine avec la protéine purifiée de *Pseudoalteromonas atlantica* (Q15XG7) (B) correspondant à une kappa-carraghénane sulfatase. L'apparition des nouveaux signaux et notamment d'un nouveau signal correspondant au proton anomérique du beta-carraghénane démontre la conversion de kappa en beta-carraghénane.

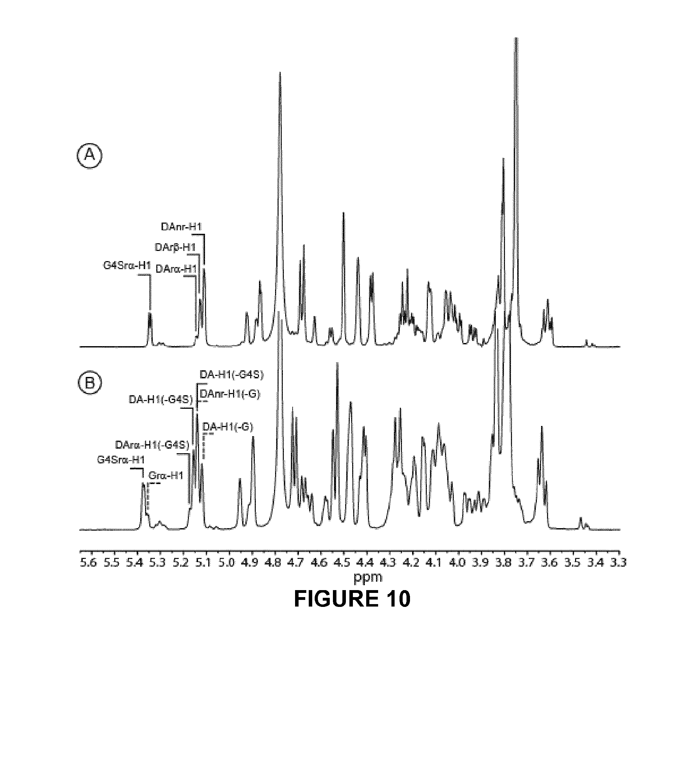

- La figure 10 représente Spectres spectre ¹H RMN d'un mélange d'oligo-kappa-carraghénane (A) incubé avec la protéine avec la protéine purifiée de *Pseudoalteromonas atlantica* (Q15XG7) (B) correspondant à une kappa-carraghénane sulfatase. L'apparition des nouveaux signaux démontre la conversion de kappa en beta-carraghénane.

### EXEMPLES

### Exemple 1 : identification et production de 4S-kappa-sulfatase selon l'invention

### Criblage des activités carraghénane-sulfatases de Pseudoalteromonas atlantica

Le criblage a été réalisé en utilisant la bactérie *Pseudoalteromonas atlantica* T6c (ATCC BAA-1087) dont le génome a été complètement séquencé (http://genome.jgi-psf.org/finished_microbes/pseat/pseat.home.html).

La production de l'extrait bactérien contenant l'activité sulfatase a été réalisée à partir de la bactérie marine *Pseudoalteromonas atlantica* (souche ATCC T6c / BAA-1087) cultivée dans du milieu ZoBell. Celui-ci a été préparé à partir de Bacto peptone (Amresco) 5g/l, d'extraits de levure (BD, Extract of Autolized Yeast cells) 1g/l, de 800ml d'eau de mer filtrée, complété à 1 litre par de l'eau déminéralisée. Afin d'induire potentiellement une activité sulfatase active sur le kappa carraghénane, les cultures ont été réalisées en présence de iota-, kappa ou lamba- carraghénane à 1g/l.

La première préculture a consisté à ensemencer 10 ml de milieu ZoBell à partir d'un glycérol de *P. atlantica* conservé à -80°C. L'incubation a été réalisée dans un agitateur de type New Brunswick pendant 36 heures à 18°C et 180 tours par minute (rpm). Pour la deuxième préculture, 50 ml de milieu ZoBell ont été ensemencés avec environ 1ml de la première préculture, de manière à avoir une absorbance de 0,1 à 660 nm. Cette deuxième préculture a été incubée à 18°C jusqu'à ce que la densité optique atteigne une valeur de 1 à 1,2 à 660 nm, soit environ 8h. Pour la culture finale, 950 ml de milieu ZoBell contenant du iota-, kappa- ou lambda-carraghénane, a été ensemencé avec les 50 ml de la deuxième préculture, et incubé à 18°C pendant 36 heures.

Afin de séparer le culot bactérien du surnageant de culture, les cultures bactériennes ont été centrifugées à 6200 g pendant 20 minutes à 4°C.

P. atlantica a été cultivé en milieu ZoBell en présence de kappa-, de iota- ou de lambda-carraghénane. La production des activités sulfatases a été évaluée en mesurant la quantité de sulfate libéré après incubation des différents carraghénanes en présence d'extraits bactériens. Les essais ont été conduits avec des surnageants de culture concentrés et des culots bactériens. Le relargage de sulfate a été suivi par chromatographie échangeuse d'anions (système Dionex). Les résultats présentés dans le Tableau 1 indiquent que la présence de iota-carraghénane dans le milieu de culture stimule la production des carraghénane-sulfatases par la bactérie. Les enzymes agissant sur le iota-carraghénane sont les plus actives et une 4S-iota-carraghénane-sulfatase a été isolée précédemment tel que décrit dans Préchoux et al (A. Préchoux, S. Genicot-Joncour, W. Helbert. Procédé de transformation du iota-carraghénane en alpha-carraghénane à l'aide d'une nouvelle classe de 4S-iota-carraghénane sulfatase. demande de brevet français n° FR 10/58420. Demande de brevet international PCT n°PCT/FR2011/052421 **[13]).** Une activité non négligeable a été détectée sur l'hybride kappa-/mu-carraghénane suggérant la présence d'une nouvelle carraghénane-sulfatase.

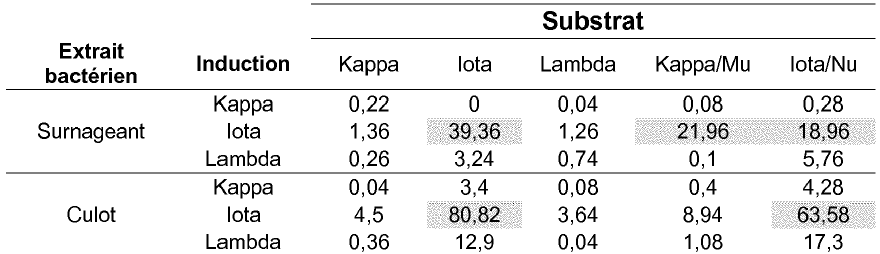

Le culot bactérien, contenant l'activité sulfatase, a été resuspendu dans du tampon Tris (Sigma) HCl 50mM à pH 7,0. Les cellules ont été ensuite lysées à la presse de French, et le lysat obtenu a été ultra-centrifugé à 27 200 g pendant 2h45. Un demi-comprimé d'anti-protéase (Complete, EDTA-free, Roche) a été ajouté au surnageant obtenu.

L'extrait, a été ensuite dialysé (Spectra/Por, MWCO 6-8000Da) contre du tampon Tris-HCl 50mM à pH 7,0 pendant une nuit, sous agitation à 4°C.

### Purification d'une nouvelle kappa-carraghénane-sulfatase

La purification de la kappa-carraghénane sulfatase a été entreprise en mettant en oeuvre différentes étapes de chromatographie. Le protocole conduisant à la plus grande pureté de l'activité sulfatase a été obtenu après un fractionnement sur colonne d'affinité Héparine Fast Flow (Hiprep 20 ml, GE Healthcare) et sur une colonne échangeuse d'anions forte Q Fast Flow (Hitrap 5 ml, GE Healthcare).

Le procédé de purification était le suivant :

Les étapes de purification ont été réalisées à l'aide d'un système Akta Purifier.

Le lysat bactérien dialysé (environ 35 ml filtré à 0,45 µm) a été chargé à l'aide d'une superloop à un débit de 2 ml/min, sur une colonne d'affinité Héparine Fast Flow (Hiprep 20 ml, GE Healthcare) préalablement équilibrée dans du tampon Tris-HCl 50 mM à pH 7,0. La résine a été ensuite lavée avec ce même tampon jusqu'à obtenir une absorbance négligeable à 280 nm. L'élution des protéines a été réalisée à un débit de 2,5 ml/min par un gradient croissant en NaCl de 0 à 1 M en 20 volumes de colonne. Les fractions collectées étaient de 3 ml et testées pour leur capacité à désulfater le kappa/mu-carraghénane. La majorité de la 4S-kappa-sulfatase a été éluée entre 1 h et 1 h30 d'élution soit entre 400 et 600 mM NaCl.

Un pool de fractions contenant un maximum d'activité sulfatase pour un minimum d'absorbance a été filtré (Cellule Amicon Bioséparations, Millipore) sur une membrane de 100 kDa puis concentré sur une autre de 30 kDa. Cet extrait, redilué dans du tampon Tris-HCl 50 mM à pH 7,5, a été alors déposé à l'aide d'une superloop sur une colonne échangeuse d'anions forte Q Fast Flow (Hitrap 5 ml, GE Healthcare) préalablement équilibrée dans le même tampon. Après lavage de la résine, les protéines ont été éluées avec un gradient croissant en NaCl de 0 à 1M en 20 volumes de colonne à un débit de 2 ml/min. Les fractions collectées, de 2,5 ml, ont été incubées en présence de kappa/micro-carraghénane pour mesurer l'activité sulfatase. Celle-ci a été éluée dès les premières minutes d'élution entre 4 et 20 min, équivalent de 160 à 380 mM NaCl. Les fractions d'intérêt ont été analysées sur gel d'électrophorèse SDS-PAGE.

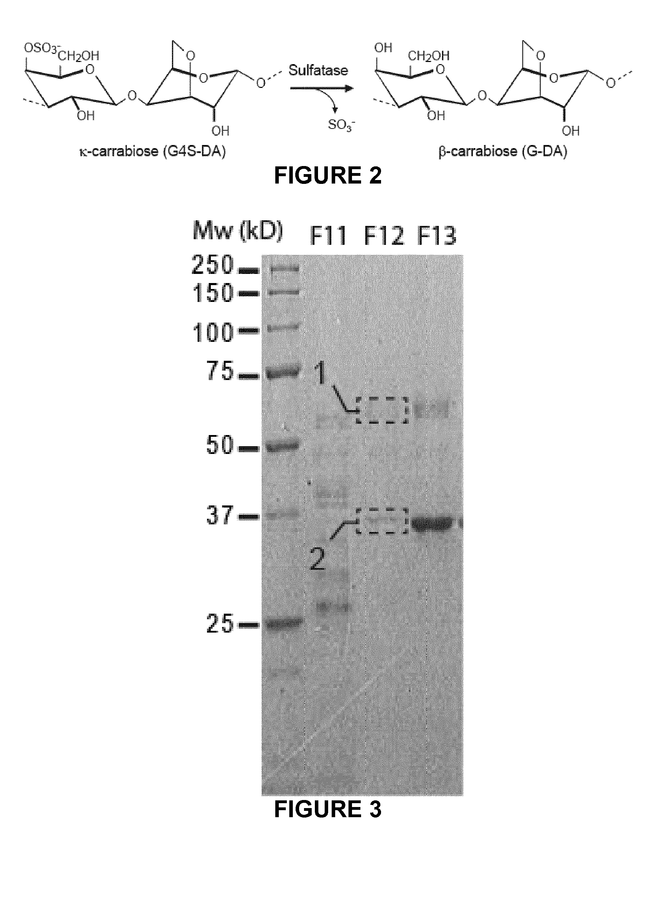

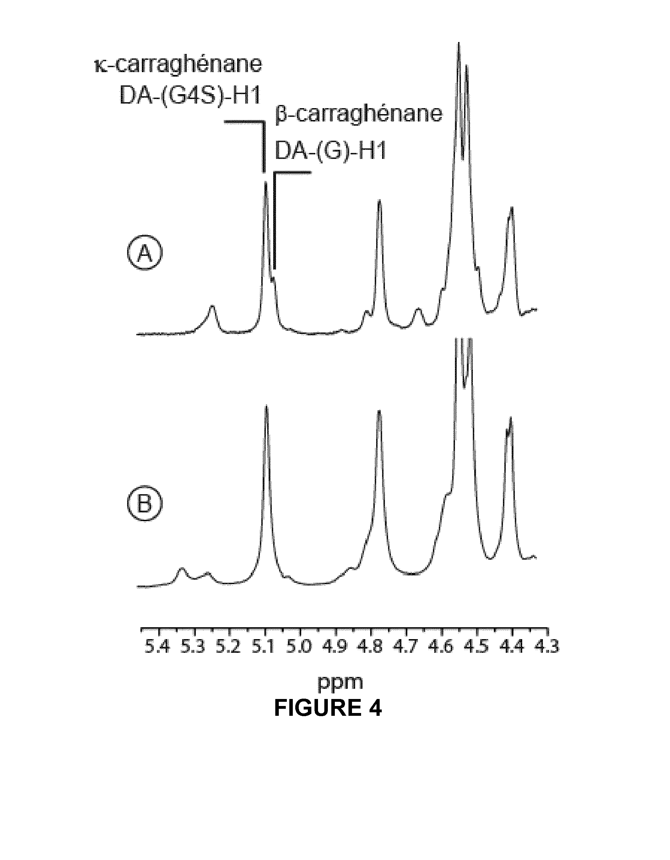

Le gel SDS-PAGE présentée à la Figure 3 a révélé que l'extrait purifié qui possède le maximum d'activité (fraction F12) et convertit le kappa- en β-carraghénane (Figure 4) contient deux fractions protéiques majoritaires à 35 kDa et 60kDa. Les deux bandes ont été excisées du gel, digérées à la trypsine et les peptides obtenus ont été séquencés par spectrométrie de masse sur la plate-forme RIO "Biopolymères" localisée à l'INRA de Nantes.

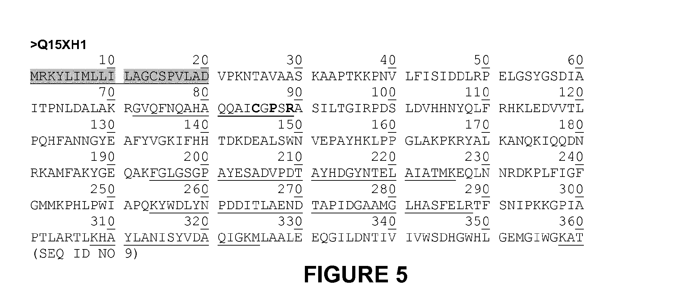

La fraction à 30kDa est composée d'une fructose-bisphosphate aldolase (Q15QK5), d'un régulateur de transcription (Q15VE2), mais pas de protéines ressemblant à une sulfatase. Par contre, la bande à 60kDa contient la protéine Q15XH1 (uniprot) de *P. atlantica* avec un poids moléculaire de 63kDa. Neuf peptides couvrant 27% de la séquence de la protéine ont été identifiés. La protéine Q15XH1 possède toutes les caractéristiques des sulfatases à Formylglycine(FGIy-sulfatase). Elle présente la séquence consensus de 12 acides aminés (C/S-X-P-S/X-R-XXX-L/X-G/X-R/X) requise pour la conversion de la cystéine en résidu Calpha-Formylglycine (FGIy) et les acides aminés catalytiques présents dans la séquence conservée G-Y/V-X-S/T-XXX-G-K-X-X-H (Figure 5).

D'autres protéines étaient également présentes dans la fraction 60kDa telles que la protéine Q15PD3 correspondant à une chaperonine.

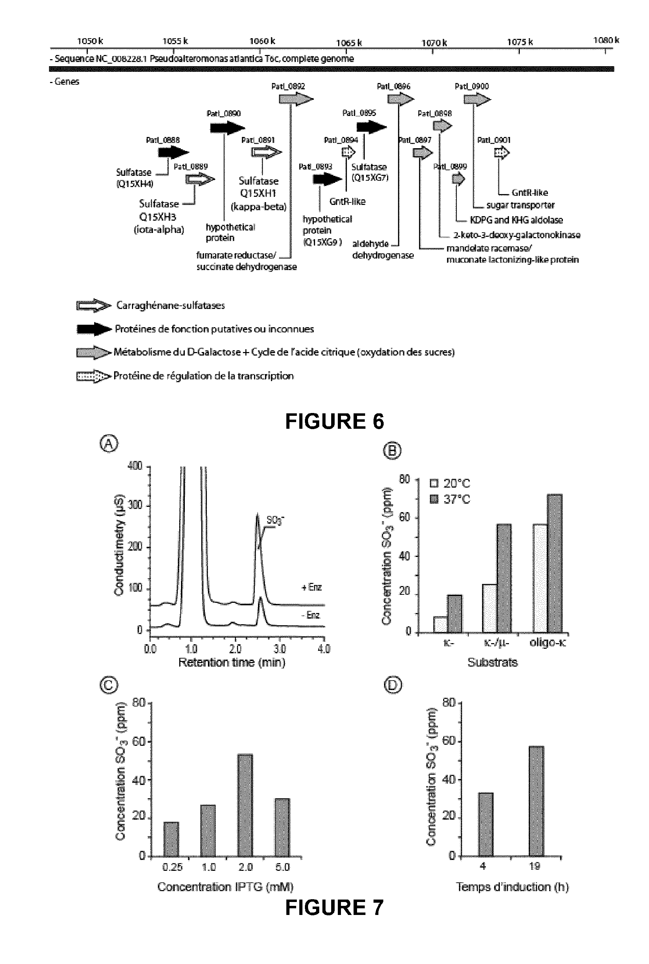

Un autre élément soutenant l'activité kappa-carraghénane-sulfatase de la protéine Q15XH1 réside dans sa localisation génomique tel que représenté sur la Figure 6. En effet, le gène de la Q15XH1 (Patl_0891) se situe dans le même cluster de gènes que celui de la iota-carraghénane-sulfatase (Q15XH3) précédemment identifiée. Ce cluster inclut plusieurs gènes du cycle citrique (oxydation des sucres) et du métabolisme du D-galactose suggérant leur rôle dans la dégradation des carraghénanes

### Optimisation des conditions d'expression de la kappa-carraghénane sulfatase active.

Le gène de la kappa-carraghénane-sulfatase a été cloné dans le plasmide Pf04 et exprimé dans *E. coli* BL21(DE3) selon les conditions décrites par Groisillier et al. (2010) (Groisillier A, Hervé C, Jeudy A, Rebuffet E, Pluchon PF, Chevolot Y, Flament D, Geslin C, Morgado IM, Power D, Branno M, Moreau H, Michel G, Boyen C, Czjzek M. 2010. MARINE-EXPRESS: taking advantage of high throughput cloning and expression strategies for the post-genomic analysis of marine organisms. Microbial Cell Factories 9: 45-56. **[14]).** Cependant, bien que *E. coli* puisse catalyser la modification post-traductionnelle de la cystéine du site actif en Calpha-formylglycine, les taux de modification sont généralement très faibles. Les conditions de culture et d'induction ont donc été optimisées pour la production d'enzyme active.

La séquence d'acides aminés obtenue correspond à la séquence de la figure 5.

### Caractérisation de l'activité sulfatase

50 µl de l'extrait bactérien ont été mis en présence de 50 µl d'une solution de substrat (oligo-carraghénane ou carraghénane) à 0,5% (p/v) dans du tampon Tris-HCl 50 mM à pH 7,5. A ce mélange a été ajouté 10 µl de CaCl₂, connu pour favoriser l'activité des sulfatases à formylglycine. Les milieux réactionnels ont été alors incubés à 30°C en bain-marie pendant une nuit. Pour chaque échantillon, un blanc a été effectué dans des conditions similaires en ayant préalablement inactivé l'extrait enzymatique à 100°C durant une quinzaine de minutes.

### Dosage du sulfate libéré

Les milieux réactionnels ont été dilués par 2 avec de l'eau milliQ (Millipore) puis centrifugés en microcons (Amicon) avec un seuil de coupure à 10kD. Cette centrifugation a été réalisée à 3300 g pendant 90min à température ambiante. Le filtrat obtenu a été ensuite dosé par chromatographie échangeuse d'anions (HPAEC : High performance anion exchange chromatography) sur un système Dionex. 20µl d'échantillon sont injectés grâce à un injecteur automatique (AS3000, Thermo). La séparation des anions présents dans les échantillons a été effectuée grâce à une colonne IonPac AS11 (4x200mm, Dionex) munie d'une pré-colonne AG-11 (4x50mm, Dionex). Le système a été équilibré en NaOH 12mM. L'élution a été réalisée par un gradient isocratique en NaOH à un débit de 1ml/min (pompe GP40, Dionex). La détection des anions a été faite par conductimétrie avec un détecteur ED40 (Dionex) muni d'un suppresseur ASRS ultra-II-4mm (Dionex) fonctionnant à un courant de 198mA. Le logiciel utilisé pour l'acquisition et le traitement des données était le logiciel Chroméléon 6.8. Grâce à une courbe étalon, l'aire des pics de sulfate a été convertie en parties par million (ppm). La différence entre la valeur de l'échantillon et celle du blanc donne la quantité de sulfate libéré en ppm lors de la réaction enzymatique de désulfatation.

L'activité sulfatase a été mesurée par dosage de la concentration des ions sulfate présents dans le milieu d'incubation par chromatographie échangeuse d'anions selon le procédé précité. Les résultats obtenus sont représenté sur la Figure 7A. L'activité kappa-carraghénane-sulfatase était la plus abondante quand E. coli était cultivée à 37°C (Figure 7B). Cette activité était la plus forte sur les oligo-kappa-carraghénanes, suivi par l'hybride kappa-/mu-carraghénane et le kappa-carraghénane. Ceci s'expliquaient par l'accessibilité de l'enzyme au substrat. La concentration d'IPTG de 2 mM et un temps d'induction de 19 h ont permis d'obtenir les meilleures activités sulfatase. Les oligos présentaient une meilleure réactivité que les polymères. Le kappa-/mu-carraghénane qui était peu gélifiant était plus désulfaté que le kappa-carraghénane standard.

La resuspension du culot bactérien après culture, ainsi que la lyse des cellules ont été réalisées dans du tampon Tris-HCl 50mM additionné de 200mM NaCl.

La sulfatase recombinante active a été fractionnée et reconcentrée par des étapes de filtration des extraits d'***E***. *coli*. Les extraits ont été filtrés après la lyse des cellules sur membrane de 100kDa. Le filtrat a été ensuite concentré sur membrane de 30kDa. Les seuils de coupure des membranes utilisés ont été sélectionnés par connaissance du poids moléculaire de la sulfatase de 63kDa.

La position du groupement sulfate clivé et donc l'identification du produit formé lors de l'hydrolyse enzymatique, a été réalisée par RMN. Pour cette analyse, les réactions de désulfatation on été faites en incubant 700µl de substrat (carraghénane ou oligo-carraghénane) à 1 % (w/v) en présence de 300µl d'extrait bactérien. Les mélanges réactionnels ont été incubés à 30°C en bain-marie durant 72h puis lyophilisés. Les échantillons ont été alors échangés deux fois dans du D20 puis redissous dans 700µl de D20 à 99,97% pour être à une concentration approximative de 10 mg/ml. Les spectres ¹H-RMN ont été enregistrés à 70°C sur un spectrophotomètre BRUKER Avance DRX 500 par le service RMN (Université de Bretagne Occidentale, Brest). Les protons anomériques des carraghénanes présentent des déplacements chimiques (δ) caractéristiques compris entre 5 et 5,6 ppm environ.

Les résultats montrent que la désulfatation du kappa-carraghénane par la protéine pure a conduit à la production de béta-carraghénane tel que représenté sur la figure 4.

Une nouvelle sulfatase capable de convertir du kappa- en beta-carraghénane a donc été identifiée.

### Example 2 : production de 4S-kappa-sulfatase selon l'invention et production de beta-carraghénane

Les procédés et matériaux utilisés dans cet exemple correspondent à ceux utilisés dans l'exemple 1.

Dans cet exemple, le gène codant pour la protéine Q15XG7 situé dans l'environnement du gène codant pour la protéine Q15XH1 (figure 6) a été cloné et surexprimé dans *E. coli.*

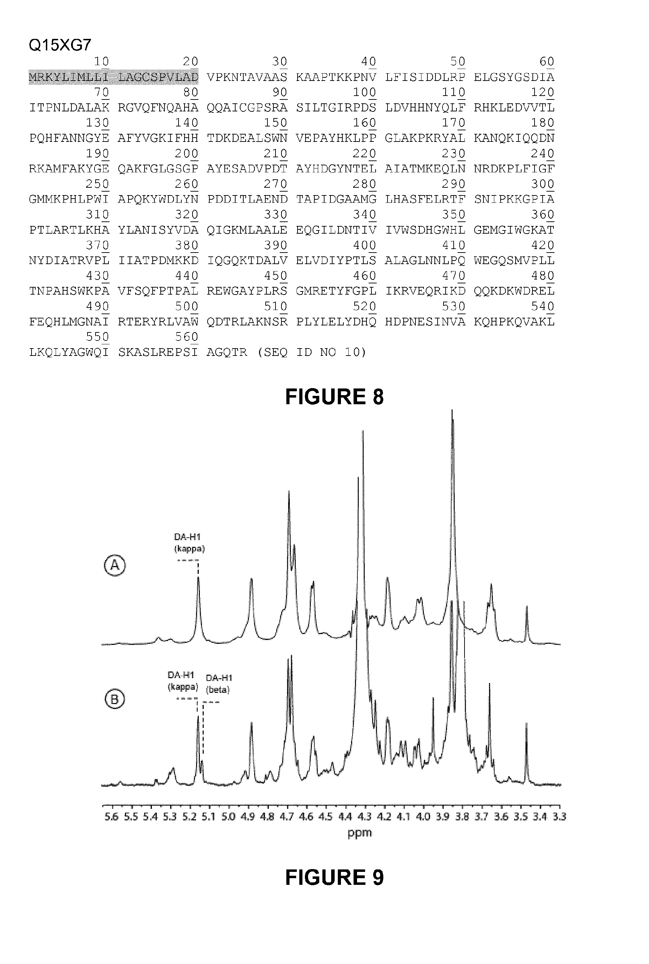

La séquence d'acides aminés obtenue correspond à la séquence de la figure 8.

### Surexpression de la sulfatase Q15XG7

La séquence du gène Patl_0895 codant pour la sulfatase Q15XG7 a été optimisée pour l'expression dans *E. coli* et synthétisée par GeneArt (Allemagne). Les gènes ont été insérés dans le vecteur d'expression pf04. La souche *E. coli* Origami 2(DE3) (Novagen, USA) a été transformée par le plasmide pF04 par choc thermique.

Une pré-culture consiste à ensemencer 10 ml de milieu LB (10 g Tryptone (Amresco), 5g Yeast Extract (Extract of Autolized Yeast cells, BD) et 10 g NaCl (Sigma) dissous dans 1 litre d'eau osmosée) à partir d'un glycérol d' *E. coli* Origami 2(DE3) contenant la sulfatase. A cette pré-culture est ajouté de l'ampicilline 10% (1µl/ml). L'incubation est réalisée une nuit à 37°C et 180 rpm. La culture a été ensuite effectuée en inoculant 100 ml de milieu LB comprenant de l'ampicilline (milieu LB + ampicilline) en Erlen de 1 L, par 1 ml de la première préculture. La culture a été alors incubée à 37°C durant 6 heures environ à 180 tours par minutes (rpm), et la densité optique du milieu a été suivie à 600 nm pour vérifier la prolifération de la bactérie. L'induction a été ensuite effectuée en ajoutant à la culture 100 ml de milieu LB + ampicilline, et de l'IPTG pour une concentration finale de 2 mM. La culture a été remise à incuber durant 19 heures à 180 rpm à 37°C.

Afin de séparer le culot bactérien du surnageant de culture, les cultures bactériennes ont été centrifugées à 6200 g pendant 25 min à 4°C. Le culot bactérien, contenant la sulfatase surexprimée, a été resuspendu dans du tampon Tris-HCl (50 mM, pH 7,5), NaCl 200 mM et ¼ de comprimé d'antiprotéase (Complete, EDTA-Free, Roche). Les cellules ont été ensuite lysées à la presse de French, et les extraits obtenus ultracentrifugés à 46 500 g pendant 2h environ.

Les extraits de sulfatases surexprimées dans *E. coli* Origami 2(DE3) ont été fractionnés par filtration sur membrane (Cellule Amicon Bioséparations, Millipore) via une ultrafiltration sur 100 kDa (dilution 5 avec le tampon de dialyse). Le filtrat récolté est ensuite reconcentré sur une membrane de 30 kDa.

### Réaction de désulfatation - production de béta-carraghénane

50 µl de l'extrait bactérien a été mis en présence de 50 µl d'une solution de substrat (oligo-kappa-carraghénane ou kappa-carraghénane) à 0,5% (p/v) dans du tampon Tris-HCl 50 mM à pH 7,5. A ce mélange a été ajouté 10 µl de CaCl₂, connu pour favoriser l'activité des sulfatases à formylglycine. Les milieux réactionnels ont été alors incubés à 30°C en bain-marie pendant une nuit.

La figure 8 représente les spectres RMN du proton du kappa-carraghénane (A) incubé avec la protéine recombinante Q15XG7 (B). L'apparition des nouveaux signaux et notamment d'un nouveau signal correspondant au proton anomérique du beta-carraghénane démontre la conversion de kappa en beta-carraghénane.

La figure 9 représente les spectres RMN d'un mélange d'oligo-kappa-carraghénane (A) incubé avec la protéine recombinante Q15XG7 (B). Comme dans le cas du polymère, les nouveaux signaux observés correspondent à la désulfatation d'unités kappa-carrabiose en beta-carrabiose. On note, que toutes les unités kappa-carrabiose sont susceptibles d'être désulfatées. En effet, les unités carrabioses internes et localisées aux extrémités réductrices et non-réductrices ont été désulfatées.

Les résultats montrent que la désulfatation du kappa-carraghénane par la protéine pure a conduit à la production de béta-carraghénane tel que représenté sur les figures 8 et 9.

Une nouvelle sulfatase capable de convertir du kappa- en beta-carraghénane a donc été identifiée.

### Listes des références

**[1]** D. Seisum (2011) Overview of the Food Hydrocolloids Market Gums and Stabilisers for the Food Industry 16, 1-8.
**[2]** Greer C, Yaphe W. (1984a) Characterization of hybrid (beta-kappa-gamma) carrageenan from Eucheuma gelatinae agardh,j. (rhodophyta, solieriaceae) using carrageenases, infrared and 13C-nuclear magnetic-resonance spectroscopy. Bot Mar 27: 473-478.
**[3]** G. Correc, A. Barabanova, R. Tuvikene, K. Truus, I. Yermak and W. Helbert, Comparison of the structures of hybrid κ-/β-carrageenans extracted from Furcellaria lumbricalis and Tichocarpus crinitus. Carbohydr. Polym. (2012) 88, 31-36.
**[4]** E. Bjerre-Petersen, J. Christensen, P. Hemmingsen (1973) Furcellaran. R.L. Whistler, J.N. BeMiller (Eds.), Industrial Gum, Academic Press, New York, pp. 123-136
**[5]** W. Zhang, L. Piculell, S. Nilsson and S. Knutsen (1994) Cation specific and cation binding to low sulfated carrageenans. Carbohydr. Polym. 23, 105-110.
**[6]** http://www.cbs.dtu.dk/services/SignaIP/
**[7]** http://bmbpcu36.leeds.ac.uk/prot_analysis/Signal.html
**[8]** http://www.promega.com/vectors/mammalian_express_vectors.htm
**[9]** http://www.qiagen.com/pendantview/qiagenes.aspx?gaw=PROTQIAge nes0807&gkw=mammalian+expression
**[10]** http://www.scbt.com/chap_exp_vectors.php?type=pCruzTM%20Expr ession%20Vectors
**[11]** WO 83/004261
**[12]** ZoBell, CE 1941 Studies on marine bacteria. I. The cultural requirements of heterotrophic aerobes, J Mar Res 4, 41-75.
**[13]** A. Préchoux, S. Genicot-Joncour, W. Helbert. Procédé de transformation du iota-carraghénane en alpha-carraghénane à l'aide d'une nouvelle classe de 4S-iota-carraghénane sulfatase. demande de brevet français n° FR 10/58420. Demande de brevet international PCT n°PCT/FR2011/052421
**[14]** Groisillier A, Hervé C, Jeudy A, Rebuffet E, Pluchon PF, Chevolot Y, Flament D, Geslin C, Morgado IM, Power D, Branno M, Moreau H, Michel G, Boyen C, Czjzek M. 2010. MARINE-EXPRESS: taking advantage of high throughput cloning and expression strategies for the post-genomic analysis of marine organisms. Microbial Cell Factories 9: 45-56.
**[15]** http://www.emdmillipore.com/life-science-research/vector-table-novagen-pet-vector-table/c_HdSb.s1077QAAAEhPqsLdcab
**[16]** http://fr-fr.invitrogen.com/site/fr/fr/home/Products-and-Services/Appl ications/Protein-Expression-and-Analysis/Protein-Expression.html

## Revendications

1. Kappa carraghénane sulfatase extraite du microorganisme *Pseudoalteromonas atlantica* T6c déposé sous le numéro ATCC T6c/BAA-1087 à l'American Type Culture Collection (ATCC).

2. Kappa carraghénane sulfatase selon la revendication 1, ladite kappa carraghénane sulfatase étant une protéine de séquence de séquence SEQ ID n°1 ou 2 du listage de séquences annexé.

3. Kappa carraghénane sulfatase selon la revendication 1 ou 2, comprenant en outre, à son extrémité N-terminale, une séquence signal.

4. Kappa carraghénane sulfatase selon l'une quelconque des revendications 1 à 3, dans laquelle la séquence signal est la séquence SEQ ID n°3 ou 4 du listage de séquences annexé.

5. Acide nucléique isolé de séquence SEQ ID n°5 ou 6 du listage de séquences annexé codant pour une kappa carraghénane sulfatase selon l'une quelconque des revendications 1 à 3.

6. Acide nucléique isolé selon la revendication 5 comprenant en outre, à son extrémité 5', la séquence SEQ ID n°7 ou 8 du listage de séquences annexé.

7. Vecteur comprenant un acide nucléique isolé selon la revendication 5 ou 6.

8. Cellule hôte comprenant une séquence d'acide nucléique isolé selon la revendication 5 ou 6 ou un vecteur selon la revendication 7.

9. Procédé de fabrication d'une protéine selon l'une quelconque des revendications 1 à 4 par recombinaison génétique utilisant un acide nucléique selon la revendication 5 ou 6 ou un vecteur selon la revendication 7.

10. Procédé de transformation de kappa-carraghénanes en béta-carraghénanes comprenant l'utilisation d'un microorganisme déposé sous le numéro ATCC T6c/BAA-1087 à American Type Culture Collection (ATCC) et/ou une kappa-carraghénane sulfatase selon l'une quelconque des revendications 1 à 4 et/ou avec une cellule hôte selon la revendication 8.

11. Procédé de transformation selon la revendication 10 comprenant l'utilisation de la kappa carraghénane sulfatase de séquence SEQ ID n°1 du listage de séquences annexé et de la kappa carraghénane sulfatase de séquence SEQ ID n°2 du listage de séquences annexé.


SEQUENCE LISTING

<110>  Centre National de la Recherche Scientifique (CNRS)
       UNIVERSITE PIERRE ET MARIE CURIE (PARIS 6)

<120>  kappa carraghénane sulfatase, procede de fabrication et
       utilisation

<130>  bnt216130ep00

<160>  10

<170>  PatentIn version 3.5

<210>  1
<211>  545
<212>  PRT
<213>  Pseudomonas atlantica

<400>  1

Val Pro Lys Asn Thr Ala Val Ala Ala Ser Lys Ala Ala Pro Thr Lys
1               5                   10                  15

Lys Pro Asn Val Leu Phe Ile Ser Ile Asp Asp Leu Arg Pro Glu Leu
            20                  25                  30

Gly Ser Tyr Gly Ser Asp Ile Ala Ile Thr Pro Asn Leu Asp Ala Leu
        35                  40                  45

Ala Lys Arg Gly Val Gln Phe Asn Gln Ala His Ala Gln Gln Ala Ile
    50                  55                  60

Cys Gly Pro Ser Arg Ala Ser Ile Leu Thr Gly Ile Arg Pro Asp Ser
65                  70                  75                  80

Leu Asp Val His His Asn Tyr Gln Leu Phe Arg His Lys Leu Glu Asp
                85                  90                  95

Val Val Thr Leu Pro Gln His Phe Ala Asn Asn Gly Tyr Glu Ala Phe
            100                 105                 110

Tyr Val Gly Lys Ile Phe His His Thr Asp Lys Asp Glu Ala Leu Ser
        115                 120                 125

Trp Asn Val Glu Pro Ala Tyr His Lys Leu Pro Pro Gly Leu Ala Lys
    130                 135                 140

Pro Lys Arg Tyr Ala Leu Lys Ala Asn Gln Lys Ile Gln Gln Asp Asn
145                 150                 155                 160

Arg Lys Ala Met Phe Ala Lys Tyr Gly Glu Gln Ala Lys Phe Gly Leu
                165                 170                 175

Gly Ser Gly Pro Ala Tyr Glu Ser Ala Asp Val Pro Asp Thr Ala Tyr
            180                 185                 190

His Asp Gly Tyr Asn Thr Glu Leu Ala Ile Ala Thr Met Lys Glu Gln
        195                 200                 205

Leu Asn Asn Arg Asp Lys Pro Leu Phe Ile Gly Phe Gly Met Met Lys
    210                 215                 220

Pro His Leu Pro Trp Ile Ala Pro Gln Lys Tyr Trp Asp Leu Tyr Asn
225                 230                 235                 240

Pro Asp Asp Ile Thr Leu Ala Glu Asn Asp Thr Ala Pro Ile Asp Gly
                245                 250                 255

Ala Ala Met Gly Leu His Ala Ser Phe Glu Leu Arg Thr Phe Ser Asn
            260                 265                 270

Ile Pro Lys Lys Gly Pro Ile Ala Pro Thr Leu Ala Arg Thr Leu Lys
        275                 280                 285

His Ala Tyr Leu Ala Asn Ile Ser Tyr Val Asp Ala Gln Ile Gly Lys
    290                 295                 300

Met Leu Ala Ala Leu Glu Glu Gln Gly Ile Leu Asp Asn Thr Ile Val
305                 310                 315                 320

Ile Val Trp Ser Asp His Gly Trp His Leu Gly Glu Met Gly Ile Trp
                325                 330                 335

Gly Lys Ala Thr Asn Tyr Asp Ile Ala Thr Arg Val Pro Leu Ile Ile
            340                 345                 350

Ala Thr Pro Asp Met Lys Lys Asp Ile Gln Gly Gln Lys Thr Asp Ala
        355                 360                 365

Leu Val Glu Leu Val Asp Ile Tyr Pro Thr Leu Ser Ala Leu Ala Gly
    370                 375                 380

Leu Asn Asn Leu Pro Gln Trp Glu Gly Gln Ser Met Val Pro Leu Leu
385                 390                 395                 400

Thr Asn Pro Ala His Ser Trp Lys Pro Ala Val Phe Ser Gln Phe Pro
                405                 410                 415

Thr Pro Ala Leu Arg Glu Trp Gly Ala Tyr Pro Leu Arg Ser Gly Met
            420                 425                 430

Arg Glu Thr Tyr Phe Gly Pro Leu Ile Lys Arg Val Glu Gln Arg Ile

435                 440                 445

Lys Asp Gln Gln Lys Asp Lys Trp Asp Arg Glu Leu Phe Glu Gln His
    450                 455                 460

Leu Met Gly Asn Ala Ile Arg Thr Glu Arg Tyr Arg Leu Val Ala Trp
465                 470                 475                 480

Gln Asp Thr Arg Leu Ala Lys Asn Ser Arg Pro Leu Tyr Leu Glu Leu
                485                 490                 495

Tyr Asp His Gln His Asp Pro Asn Glu Ser Ile Asn Val Ala Lys Gln
            500                 505                 510

His Pro Lys Gln Val Ala Lys Leu Leu Lys Gln Leu Tyr Ala Gly Trp
        515                 520                 525

Gln Ile Ser Lys Ala Ser Leu Arg Glu Pro Ser Ile Ala Gly Gln Thr
    530                 535                 540

Arg
545

<210>  2
<211>  446
<212>  PRT
<213>  pseudomonas atlantica

<400>  2

Gln Pro Asn Ile Val Phe Leu Phe Ser Asp Asp Ala Gly Tyr Ala Asp
1               5                   10                  15

Phe Gly Phe Gln Gly Ser Glu Thr Met Lys Thr Pro Asn Leu Asp Gln
            20                  25                  30

Leu Ala Ser Glu Gly Val Arg Phe Thr Gln Gly Tyr Val Ser Asp Ser
        35                  40                  45

Thr Cys Gly Pro Ser Arg Ala Gly Ile Met Thr Gly Arg Tyr Gln Gln
    50                  55                  60

Lys Phe Gly Tyr Glu Glu Ile Asn Val Pro Gly Tyr Met Ser Glu His
65                  70                  75                  80

Ser Ala Ile Lys Gly Ala Glu Met Gly Ile Pro Leu Asp Glu Val Thr
                85                  90                  95

Met Gly Asp Tyr Met Lys Ser Leu Gly Tyr Arg Thr Ala Phe Tyr Gly
            100                 105                 110

Lys Trp His Leu Gly Gly Thr Asp Glu Leu His Pro Met His Arg Gly
        115                 120                 125

Phe Asp Glu Phe Tyr Gly Phe Arg Gly Gly Asp Arg Ser Tyr Trp Ala
    130                 135                 140

Tyr Glu Val Asn Ala Pro Glu Arg Lys Ser Ala Val Phe Thr Asp Lys
145                 150                 155                 160

Lys Leu Glu His Gly Ile Asp Gln Phe Gln Glu His Glu Gly Tyr Leu
                165                 170                 175

Thr Asp Val Leu Ala Glu Lys Ala Asn Gln Phe Ile Glu Lys Ala Pro
            180                 185                 190

Asp Lys Pro Phe Phe Ile Phe Leu Ser Phe Asn Ala Val His Thr Pro
        195                 200                 205

Met Glu Ala Thr Pro Glu Asp Leu Ala Lys Phe Pro Gln Leu Lys Gly
    210                 215                 220

Lys Arg Lys Glu Val Ala Ala Met Thr Leu Ala Leu Asp Arg Ala Ser
225                 230                 235                 240

Gly Ala Val Leu Asn Lys Leu Lys Glu Leu Gly Leu Glu Asp Asp Thr
                245                 250                 255

Leu Val Val Phe Ser Asn Asp Asn Gly Gly Pro Thr Asp Lys Asn Ala
            260                 265                 270

Ser Ser Asn Tyr Pro Leu Ala Gly Thr Lys Ser Asn Phe Leu Glu Gly
        275                 280                 285

Gly Ile Arg Val Pro Phe Leu Val Lys Trp Pro Ala Lys Leu Ala Ala
    290                 295                 300

Gly Lys Val Tyr Asp Lys Pro Val Ser Thr Leu Asp Leu Leu Pro Thr
305                 310                 315                 320

Phe Phe Lys Ala Gly Gly Gly Glu Glu Val Met Ser Glu Leu Asp Gly
                325                 330                 335

Val Asp Leu Met Pro Tyr Ile Thr Gly Gln Asn Asn Lys Ala Pro His
            340                 345                 350

Glu Ser Met Tyr Trp Lys Lys Glu Thr Arg Ala Ala Ile Arg Gln Gly
        355                 360                 365

Asp Trp Lys Leu Leu Arg Phe Pro Asp Arg Pro Ala Glu Leu Tyr Asn
    370                 375                 380

Leu Ala Asn Asp Ile Gly Glu Gln His Asn Leu Ala Ala Gln Glu Pro
385                 390                 395                 400

Glu Arg Val Lys Gln Met Tyr Lys Asp Phe Phe Ser Trp Glu Met Thr
                405                 410                 415

Leu Glu Arg Pro Leu Trp Leu Leu Lys Arg Gln Phe Glu Glu Tyr Asp
            420                 425                 430

Leu Asn Arg Met Asp Lys Tyr Arg Val Pro Lys Arg Leu Glu
        435                 440                 445

<210>  3
<211>  20
<212>  PRT
<213>  Artificial Sequence

<220>
<223>  séquence signal

<400>  3

Met Arg Lys Tyr Leu Ile Met Leu Leu Ile Leu Ala Gly Cys Ser Pro
1               5                   10                  15

Val Leu Ala Asp
            20

<210>  4
<211>  25
<212>  PRT
<213>  Artificial Sequence

<220>
<223>  séquence signal

<400>  4

Met Lys Ser Val Phe His Arg Val Val Ala Ala Leu Ser Ile Ser Val
1               5                   10                  15

Ala Cys Thr Ser Leu Ser Tyr Ala Lys
            20                  25

<210>  5
<211>  1638
<212>  DNA
<213>  Artificial Sequence

<220>
<223>  séquence d'acides nucléiques codant pour une
       kappa-carraghénane-sulfatase

<400>  5
gtaccaaaga acactgccgt agctgctagc aaagctgcac caactaaaaa gccaaatgta      60

ttatttatct cgattgatga cttgcgccct gagctgggca gttacggttc tgatattgcc     120

ataacaccca atttagatgc tttggcgaag cgcggcgtac agtttaatca agctcatgca     180

cagcaagcga tatgtgggcc atctcgcgcc agcatattaa caggcattcg acctgattca     240

cttgatgtac atcataatta tcaattgttc agacataaac ttgaggacgt ggttaccctg     300

cctcagcatt ttgctaacaa tggttatgaa gcattttacg tgggtaaaat cttccatcac     360

accgacaaag acgaagcact gtcttggaat gttgagcccg cttatcacaa gctacctcct     420

gggttagcaa aacccaaacg ttatgcatta aaagccaacc aaaaaattca gcaagataac     480

cgtaaggcta tgttcgccaa gtacggtgaa caagctaaat ttggcttagg tagtgggcca     540

gcatatgaat ctgcagacgt gccagatacg gcctaccacg acggttataa caccgagctt     600

gccattgcta cgatgaaaga gcagttgaat aatcgtgata agcctttgtt tatcggattt     660

gggatgatga agccgcactt gccatggatc gcgccgcaaa aatattggga cctttataac     720

ccagatgaca ttactctagc tgagaacgac acagcgccta ttgatggcgc agcaatgggt     780

ttacacgcat cttttgaact tcgcactttt tccaatattc ccaaaaaagg gcctatagca     840

ccgactctag cgcgcacact caagcatgct tatttagcca acatcagtta tgtggacgcg     900

cagattggga aaatgcttgc agcccttgaa gagcaaggga tcttagataa taccatcgtg     960

atcgtgtgga gtgaccatgg ctggcactta ggcgaaatgg gtatctgggg caaagccacg    1020

aactacgata tagccacccg agttccgctt attattgcca cgccagatat gaaaaaagac    1080

attcaagggc aaaaaacaga tgctctggtc gaactggtcg atatttatcc gacgttgtca    1140

gcacttgcag gcttgaataa cttaccccaa tgggaagggc aaagcatggt gccgcttttg    1200

accaatcctg ctcactcttg gaaaccggct gtgtttagtc aatttcctac gccagcattg    1260

agagaatggg gggcttatcc gctgcgttct ggtatgcggg aaacttactt cgggccgctt    1320

attaaacgag ttgaacagcg catcaaagac caacaaaaag acaaatggga tcgagaacta    1380

tttgaacaac acttgatggg caatgctata cgtaccgagc gatatcggct ggtggcttgg    1440

caagataccc gtttagctaa gaatagccgt cctttgtact tagagttgta tgatcatcaa    1500

catgatccta acgaaagcat caatgttgcc aaacaacacc caaaacaagt ggccaaacta    1560

ctcaaacaac tctatgcggg ttggcaaatc agtaaagcgt cattaagaga accctcaatc    1620

gcaggacaaa cacgatga                                                  1638

<210>  6
<211>  1341
<212>  DNA
<213>  Artificial Sequence

<220>
<223>  séquence d'acides nucléique codant pour une kappa-carraghénane

sulfatase

<400> 6
caaccgaata tcgtttttct gttttccgat gatgcagggt atgctgactt tggttttcaa 60

ggcagcgaaa caatgaaaac acccaatttg gatcagcttg ctagcgaagg tgttagattt 120

acccaaggtt atgtgtcgga ctctacttgc ggcccatctc gggcgggcat catgacaggg 180

cgctaccagc aaaaattcgg ttacgaagaa ataaacgtgc cgggttatat gagtgagcac 240

tctgctataa aaggggcaga aatggggatc ccgttagatg aagtcactat gggtgactac 300

atgaaatctc ttgggtatcg cacggcgttc tacggtaaat ggcacttagg tggtacggat 360

gaattgcacc ctatgcaccg tggcttcgac gaattttatg gctttcgcgg cggcgatcgc 420

agctactggg cctatgaagt caatgcccct gagcgcaaat ctgccgtatt tactgacaaa 480

aaacttgaac acggtattga tcaatttcaa gagcacgaag ggtatttgac cgatgtgtta 540

gccgagaaag ccaatcaatt tattgaaaaa gcgcctgata aaccgttctt tattttctta 600

tcatttaacg cagtgcatac accaatggaa gccacgccag aagatttagc gaagtttcct 660

cagttaaaag gcaagcgcaa ggaagttgca gccatgaccc tcgcacttga tagagccagc 720

ggagcagtgc taaacaaact caaggagctg ggtttggaag atgacactct tgtggtgttc 780

agcaacgaca atggcggccc tacggataaa aacgcctcat ctaactaccc attagcaggg 840

actaaatcaa actttttgga agggggaata cgcgtgcctt ttctggttaa atggcctgct 900

aagctagctg ctggaaaggt atacgataaa cccgtcagta cgctcgactt attacccaca 960

ttctttaaag cgggtggcgg tgaagaggtg atgtctgaat tggatggcgt ggatttgatg 1020

ccttatataa caggccaaaa taacaaggca cctcacgaga gcatgtactg gaaaaaagaa 1080

actcgcgcag ctattcgcca aggagattgg aagttattgc gcttcccaga ccgtcctgca 1140

gaattgtata accttgctaa tgacattggt gaacaacata acttagccgc ccaagagcct 1200

gagcgtgtta agcaaatgta caaagatttc ttcagttggg aaatgacact agagcgccca 1260

ttatggttgc ttaagcgcca gtttgaggaa tacgatttaa atcgcatgga caagtaccgc 1320

gtaccaaaac gcttagaata a 1341

<210> 7
<211> 60
<212> DNA
<213> Artificial Sequence

<220>
<223> séquence d'acide nucléique codant pour un peptide signal

<400> 7
atgcgtaaat atttaataat gttactgatc ttggctggtt gttcacctgt gcttgctgat 60

<210> 8
<211> 75
<212> DNA

<213>  Artificial Sequence

<220>
<223>  séquence d'acide nucléique codant pour un peptide signal

<400>  8
atgaaatcag tttttcatcg cgttgtagca gcgctttcta tcagtgtggc gtgcacttcg      60

ttaagttacg ccaag                                                        75


<210>  9
<211>  360
<212>  PRT
<213>  pseudomonas atlantica

<400>  9

Met Arg Lys Tyr Leu Ile Met Leu Leu Ile Leu Ala Gly Cys Ser Pro
1               5                   10                  15

Val Leu Ala Asp Val Pro Lys Asn Thr Ala Val Ala Ala Ser Lys Ala
            20                  25                  30

Ala Pro Thr Lys Lys Pro Asn Val Leu Phe Ile Ser Ile Asp Asp Leu
        35                  40                  45

Arg Pro Glu Leu Gly Ser Tyr Gly Ser Asp Ile Ala Ile Thr Pro Asn
    50                  55                  60

Leu Asp Ala Leu Ala Lys Arg Gly Val Gln Phe Asn Gln Ala His Ala
65                  70                  75                  80

Gln Gln Ala Ile Cys Gly Pro Ser Arg Ala Ser Ile Leu Thr Gly Ile
                85                  90                  95

Arg Pro Asp Ser Leu Asp Val His His Asn Tyr Gln Leu Phe Arg His
            100                 105                 110

Lys Leu Glu Asp Val Val Thr Leu Pro Gln His Phe Ala Asn Asn Gly
        115                 120                 125

Tyr Glu Ala Phe Tyr Val Gly Lys Ile Phe His His Thr Asp Lys Asp
    130                 135                 140

Glu Ala Leu Ser Trp Asn Val Glu Pro Ala Tyr His Lys Leu Pro Pro
145                 150                 155                 160

Gly Leu Ala Lys Pro Lys Arg Tyr Ala Leu Lys Ala Asn Gln Lys Ile
                165                 170                 175

Gln Gln Asp Asn Arg Lys Ala Met Phe Ala Lys Tyr Gly Glu Gln Ala
            180                 185                 190

Lys Phe Gly Leu Gly Ser Gly Pro Ala Tyr Glu Ser Ala Asp Val Pro
        195                 200                 205

Asp Thr Ala Tyr His Asp Gly Tyr Asn Thr Glu Leu Ala Ile Ala Thr
    210                 215                 220

Met Lys Glu Gln Leu Asn Asn Arg Asp Lys Pro Leu Phe Ile Gly Phe
225                 230                 235                 240

Gly Met Met Lys Pro His Leu Pro Trp Ile Ala Pro Gln Lys Tyr Trp
                245                 250                 255

Asp Leu Tyr Asn Pro Asp Asp Ile Thr Leu Ala Glu Asn Asp Thr Ala
            260                 265                 270

Pro Ile Asp Gly Ala Ala Met Gly Leu His Ala Ser Phe Glu Leu Arg
        275                 280                 285

Thr Phe Ser Asn Ile Pro Lys Lys Gly Pro Ile Ala Pro Thr Leu Ala
    290                 295                 300

Arg Thr Leu Lys His Ala Tyr Leu Ala Asn Ile Ser Tyr Val Asp Ala
305                 310                 315                 320

Gln Ile Gly Lys Met Leu Ala Ala Leu Glu Glu Gln Gly Ile Leu Asp
                325                 330                 335

Asn Thr Ile Val Ile Val Trp Ser Asp His Gly Trp His Leu Gly Glu
            340                 345                 350

Met Gly Ile Trp Gly Lys Ala Thr
        355                 360

<210> 10
<211> 565
<212> PRT
<213> pseudomonas atlantica

<400> 10

Met Arg Lys Tyr Leu Ile Met Leu Leu Ile Leu Ala Gly Cys Ser Pro
1               5                   10                  15

Val Leu Ala Asp Val Pro Lys Asn Thr Ala Val Ala Ala Ser Lys Ala
            20                  25                  30

Ala Pro Thr Lys Lys Pro Asn Val Leu Phe Ile Ser Ile Asp Asp Leu
        35                  40                  45

Arg Pro Glu Leu Gly Ser Tyr Gly Ser Asp Ile Ala Ile Thr Pro Asn

50                  55                  60

Leu Asp Ala Leu Ala Lys Arg Gly Val Gln Phe Asn Gln Ala His Ala
65                  70                  75                  80

Gln Gln Ala Ile Cys Gly Pro Ser Arg Ala Ser Ile Leu Thr Gly Ile
                85                  90                  95

Arg Pro Asp Ser Leu Asp Val His His Asn Tyr Gln Leu Phe Arg His
            100                 105                 110

Lys Leu Glu Asp Val Val Thr Leu Pro Gln His Phe Ala Asn Asn Gly
        115                 120                 125

Tyr Glu Ala Phe Tyr Val Gly Lys Ile Phe His His Thr Asp Lys Asp
    130                 135                 140

Glu Ala Leu Ser Trp Asn Val Glu Pro Ala Tyr His Lys Leu Pro Pro
145                 150                 155                 160

Gly Leu Ala Lys Pro Lys Arg Tyr Ala Leu Lys Ala Asn Gln Lys Ile
                165                 170                 175

Gln Gln Asp Asn Arg Lys Ala Met Phe Ala Lys Tyr Gly Glu Gln Ala
            180                 185                 190

Lys Phe Gly Leu Gly Ser Gly Pro Ala Tyr Glu Ser Ala Asp Val Pro
        195                 200                 205

Asp Thr Ala Tyr His Asp Gly Tyr Asn Thr Glu Leu Ala Ile Ala Thr
    210                 215                 220

Met Lys Glu Gln Leu Asn Asn Arg Asp Lys Pro Leu Phe Ile Gly Phe
225                 230                 235                 240

Gly Met Met Lys Pro His Leu Pro Trp Ile Ala Pro Gln Lys Tyr Trp
                245                 250                 255

Asp Leu Tyr Asn Pro Asp Asp Ile Thr Leu Ala Glu Asn Asp Thr Ala
            260                 265                 270

Pro Ile Asp Gly Ala Ala Met Gly Leu His Ala Ser Phe Glu Leu Arg
        275                 280                 285

Thr Phe Ser Asn Ile Pro Lys Lys Gly Pro Ile Ala Pro Thr Leu Ala
    290                 295                 300

Arg Thr Leu Lys His Ala Tyr Leu Ala Asn Ile Ser Tyr Val Asp Ala
305                 310                 315                 320

Gln Ile Gly Lys Met Leu Ala Ala Leu Glu Glu Gln Gly Ile Leu Asp
                325                 330                 335

Asn Thr Ile Val Ile Val Trp Ser Asp His Gly Trp His Leu Gly Glu
            340                 345                 350

Met Gly Ile Trp Gly Lys Ala Thr Asn Tyr Asp Ile Ala Thr Arg Val
        355                 360                 365

Pro Leu Ile Ile Ala Thr Pro Asp Met Lys Lys Asp Ile Gln Gly Gln
    370                 375                 380

Lys Thr Asp Ala Leu Val Glu Leu Val Asp Ile Tyr Pro Thr Leu Ser
385                 390                 395                 400

Ala Leu Ala Gly Leu Asn Asn Leu Pro Gln Trp Glu Gly Gln Ser Met
                405                 410                 415

Val Pro Leu Leu Thr Asn Pro Ala His Ser Trp Lys Pro Ala Val Phe
            420                 425                 430

Ser Gln Phe Pro Thr Pro Ala Leu Arg Glu Trp Gly Ala Tyr Pro Leu
        435                 440                 445

Arg Ser Gly Met Arg Glu Thr Tyr Phe Gly Pro Leu Ile Lys Arg Val
    450                 455                 460

Glu Gln Arg Ile Lys Asp Gln Gln Lys Asp Lys Trp Asp Arg Glu Leu
465                 470                 475                 480

Phe Glu Gln His Leu Met Gly Asn Ala Ile Arg Thr Glu Arg Tyr Arg
                485                 490                 495

Leu Val Ala Trp Gln Asp Thr Arg Leu Ala Lys Asn Ser Arg Pro Leu
            500                 505                 510

Tyr Leu Glu Leu Tyr Asp His Gln His Asp Pro Asn Glu Ser Ile Asn
        515                 520                 525

Val Ala Lys Gln His Pro Lys Gln Val Ala Lys Leu Leu Lys Gln Leu
    530                 535                 540

Tyr Ala Gly Trp Gln Ile Ser Lys Ala Ser Leu Arg Glu Pro Ser Ile
545                 550                 555                 560

Ala Gly Gln Thr Arg
                565